# FASCICULE DE BREVET EUROPEEN

(11) **EP 4 061 322 B1**
(45) Date de publication et mention de la délivrance du brevet: **22.07.2026**
(21) Numéro de dépôt: 20821345.4
(22) Date de dépôt: 19.11.2020
(51) Int. Cl.: A61K 8/49, A61K 31/357, A61P 17/18, A61Q 17/04, A61Q 19/02

(54) **SYNTHESE DE DERIVES DE L'ACIDE DE MELDRUM PRESENTANT DES ACTIVITES ANTI-UVA ET LUMIERE BLEUE**
SYNTHESE VON MELDRUM-SÄURE-DERIVATEN ALS UVA- UND BLAULICHTSCREENINGMITTEL
SYNTHESIS OF MELDRUM'S ACID DERIVATIVES FOR USE AS ANTI UVA AND ANTI-BLUE LIGHT AGENT

(30) Priorité: 20.11.2019 FR 1912971
(43) Date de publication de la demande: 28.09.2022
(73) Titulaire: Institut des Sciences et Industries du Vivant et de l'Environnement - AgroParisTech, 75231 Paris Cedex 05 (FR)
(72) Inventeur: ALLAIS, Florent, 51400 BOUY (FR); PEYROT, Cédric, 51100 REIMS (FR); MENTION, Matthieu, 51450 BETHENY (FR)
(74) Mandataire: IP Trust
(86) Numéro de dépôt international: PCT/FR2020/052125
(87) Numéro de publication internationale: WO 2021/099742

(56) Documents cités:
- EP-A1- 1 433 469
- EP-A1- 3 441 115
- WO-A1-2018/083344
- DIAZ YVONNE J. ET AL: "A Versatile and Highly Selective Colorimetric Sensor for the Detection of Amines", CHEMISTRY A EUROPEAN JOURNAL, vol. 23, no. 15, 20 February 2017 (2017-02-20), DE, pages 3562 - 3566, XP055772782, ISSN: 0947-6539, Retrieved from the Internet <URL:https://api.wiley.com/onlinelibrary/tdm/v1/articles/10.1002%2Fchem.201700368> DOI: 10.1002/chem.201700368
- MIERINA INESE ET AL: "Novel type of carbon-centered antioxidants arylmethyl Meldrum's acids -inhibit free radicals", vol. 119, no. 11, 1 November 2017 (2017-11-01), pages 1700172 - n/a, XP009525436, ISSN: 1438-7697, Retrieved from the Internet <URL:https://api.wiley.com/onlinelibrary/tdm/v1/articles/10.1002%2Fejlt.201700172> [retrieved on 20170602], DOI: 10.1002/EJLT.201700172
- DUMAS ET AL: "A general and practical preparation of alkylidene Meldrum's acids", TETRAHEDRON LETTERS, ELSEVIER, AMSTERDAM , NL, vol. 48, no. 40, 1 October 2007 (2007-10-01), pages 7072 - 7074, XP022230681, ISSN: 0040-4039, DOI: 10.1016/J.TETLET.2007.08.012

## Description

L'invention se rapporte au domaine des molécules présentant des propriétés anti-UVA et anti lumière bleue.

Plus particulièrement, l'invention concerne l'utilisation de dérivés de l'acide de Meldrum présentant des activités anti-UVA et lumière bleue. Ces composés peuvent être utilisés dans de nombreux domaines d'application tels que la cosmétique notamment les crèmes solaires, l'agroalimentaire, la pharmacie, les polymères, les produits phytosanitaires et la protection des plantes contre le froid.

### Domaine de l'invention

Les filtres solaires sont constitués de molécules capables d'absorber les UV. Parmi les filtres UVA les plus efficaces et les plus utilisés dans les crèmes solaires, on trouve l'Avobenzone. Cette molécule est cependant actuellement vivement critiquée à cause de son impact environnemental (destruction des coraux) mais également pour des risques de santé publique (perturbateur endocrinien).

L'acide Kojique est un agent anti-pigmentant utilisé en tant que principe actif dans les crèmes cosmétiques notamment pour éliminer les tâches pigmentaires, les tâches de sénescence et/ou blanchir la peau. Il agit en réduisant l'activité de la tyrosinase, enzyme impliquée dans la fabrication de la mélanine. Il peut être irritant et présente un risque pour la santé du consommateur dès qu'il est utilisé à une dose supérieure à 0,1% dans un produit de soin, selon le comité scientifique européen sur les produits de consommation (SCCP).

Les molécules anti-oxydantes de référence, telles que le BHA et le BHT, très couramment utilisées dans les formulations cosmétiques, sont également controversées pour leur effet potentiellement cancérigène.

Des recherches sont menées pour proposer des molécules à usage de filtre solaire ou d'agent anti-pigmentant et/ou dépigmentant à faible impact environnemental et sur la santé. Toutefois, les molécules biosourcées développées jusqu'à présent ne sont pas stables et/ou peu efficaces.

MIERINA INESE ET AL ( "Novel type of carbon-centered antioxidants arylmethyl Meldrum's acids -inhibit free radicals",vol. 119, no. 11 1 novembre 2017, Eur,J.Sci.Technol.) dévoile l'utilisation de dérivés de l'acide de Meldrum comme agent anti oxidant et anti radicalaire

Il existe un besoin de disposer de molécules non toxiques pour la peau et l'environnement en remplacement des molécules de référence utilisées à ce jour.

### Exposé de l'invention

Les inventeurs ont mis en évidence les propriétés anti-UVA et anti-pigmentantes de dérivés de l'acide de Meldrum et proposent leur utilisation dans les différents domaines d'application où elles sont recherchées tels que la cosmétique, l'agroalimentaire, l'emballage, les produits phytosanitaires la pharmacie et les polymères.

Ainsi, l'invention concerne l'utilisation d'au moins un dérivé de l'acide de Meldrum de formule (I) 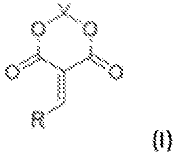 dans laquelle R est un groupement de type furane, de type phénol, de type pyrrole, de type vinylphénol ou de type vinylfurane, pour son utilisation pour la peau en tant que filtre UV-A et lumière bleue.

Ces composés peuvent être utilisés seuls ou en mélange afin de fournir une composition aux propriétés améliorées, par exemple combinant propriétés anti-UVA et anti-lumière bleue.

### Avantages de l'invention

Les dérivés de l'acide de Meldrum de formule (I) constituent une nouvelle classe de molécules biosourcées utilisables en tant que filtre UVA et lumière bleue.

Utiliser ces composés plutôt que les molécules de référence est avantageux pour plusieurs raisons.

Tout d'abord et de manière remarquable, ils présentent des propriétés d'absorption des UVA et de photostabilité équivalentes à celles d'une molécule de référence telle que l'Avobenzone, sans en avoir les inconvénients. Notamment, les inventeurs ont montré l'absence de perturbation du système endocrinien ; les tests réalisés n'ont pas mis en évidence d'interaction ni positive, ni négative avec les récepteurs PXR, hERα, hAR.

Ces dérivés sont préparés via un procédé ne mettant pas en œuvre de solvant organique, mais seulement de l'eau. Les composés sont obtenus sans étape de purification par chromatographie, souvent coûteuse en énergie et en solvant. Ils sont purifiés, soit par filtration lorsque le produit précipite, soit via une simple extraction liquide/liquide. Ce procédé est donc simple, rapide et écologique et de ce fait industrialisable.

Ces composés présentent, en fonction du type de substituant choisi, des propriétés complémentaires à celle d'un filtre UVA, telles que des propriétés anti-oxydantes et/ou anti-pigmentantes et/ou dépigmentantes.

Ils peuvent être utilisés seuls, ou en mélange entre eux ou avec d'autres types de composés, notamment des composés présentant des capacités d'absorption des UVB et/ou des propriétés anti-oxydantes et/ou anti-pigmentantes et/ou dépigmentantes. De manière tout à fait intéressante, ils peuvent notamment être associés à des dérivés de sinapoyl malate ou des dimères β-β d'analogues d'esters sinapique.

### DESCRIPTION DETAILLEE DE L'INVENTION

Un premier objet de l'invention concerne un dérivé de l'acide de Meldrum de formule (I) 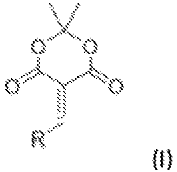 dans laquelle R est un groupement de type furane, de type phénol, de type pyrrole, de type vinylphénol ou de type vinylfurane, pour son utilisation en tant que filtre UV-A et lumière bleue.

Dans un premier mode de réalisation, le groupement R est de type furane et répond à la formule (II) 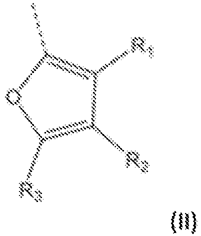 dans laquelle :
R₁ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle ;
R₁ et R₂ peuvent être combinés pour former un cycle de type -(CH₂)ₙ- ou -(CH)ₙ- avec n allant de 4 à 8
R₂ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle ;
R₁ et R₂ peuvent être combinés pour former un cycle de type -(CH₂)ₙ- ou -(CH)ₙ- avec n allant de 4 à 8
R₂ et R₃ peuvent être combinés pour former un cycle de type -(CH₂)ₙ- ou -(CH)ₙ- avec n allant de 4 à 8
R₃ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle;
R₂ et R₃ peuvent être combinés pour former un cycle de type -(CH₂)ₙ- ou -(CH)ₙ- avec n allant de 4 à 8.

Des exemples de dérivés furaniques de l'acide de Meldrum sont les composés 1 à 7 représentés ci-dessous : 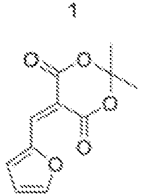 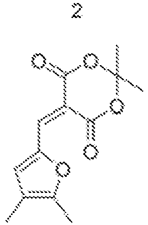 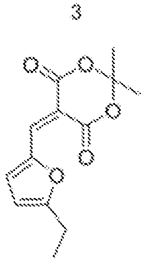 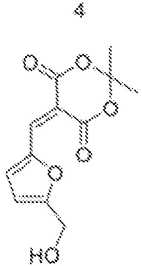 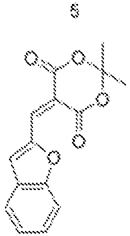 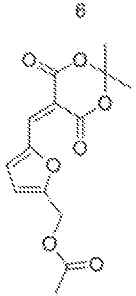 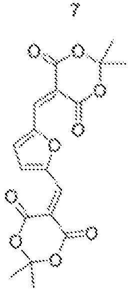 à savoir le 5-(2-Furanylmethylene)-2,2-dimethyl-1,3-dioxane-4,6-dione (composé 1), le 5-[(4,5-Dimethyl-2-furanyl)methylene]-2,2-dimethyl-1,3-dioxane-4,6-dione (composé 2), le 5-[(5-Ethyl-2-furanyl)methylene]-2,2-dimethyl-1,3-dioxane-4,6-dione (composé 3), le 5-[[5-(Hydroxymethyl)-2-furanyl]methylene]-2,2-dimethyl-1,3-dioxane-4,6-dione (composé 4), le 5-(2-benzofuranylmethylene)-2,2-dimethyl-1,3-dioxane-4,6-dione (composé 5), le 5-[[5-((Acetyloxy)methyl)-2-furanyl]methylene]-2,2-dimethyl-1,3-dioxane-4,6-dione (composé 6) et le 5,5'-(2,5-furandiyldimethylidyne)bis-2,2-dimethyl-1,3-dioxane-4,6-dione (composé 7).

Dans un mode de réalisation préféré, les composés utilisés pour leurs propriétés anti-UV-A sont les composés 1, 2, 3 et 4.

En plus des propriétés de filtre anti-UVA et lumière bleue, les composés de la série furanique présenteraient des propriétés anti-pigmentantes et/ou dépigmentantes.

Dans un second mode de réalisation, le groupement R est de type phénol et répond à la formule (III) 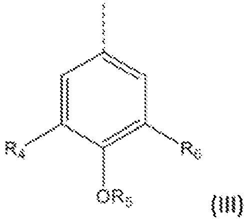 dans laquelle :
R₄ et R₆ sont indépendamment choisis parmi, un H, un halogène, ou un groupement alkyle en C₁ à C₈ linéaire, cyclique ou branché, saturé ou insaturé, ou un groupement O-alkyle en C₁ à C₈ linéaire, cyclique ou branché, saturé ou insaturé ; et
R₅ est un H, ou un groupement acétyle, ou un groupement alkyle en C₁ à C₈ linéaire, cyclique ou branché, saturé ou insaturé, ou un groupe protecteur de type silyle, époxyde, benzyle ou benzoyle.

Des exemples de dérivés phénoliques de l'acide de Meldrum sont les composés 8 à 11 représentés ci-dessous : 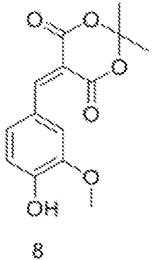 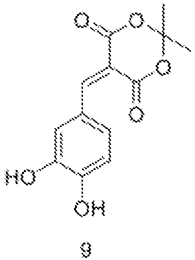 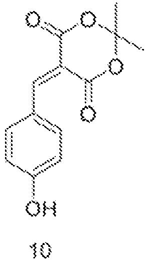 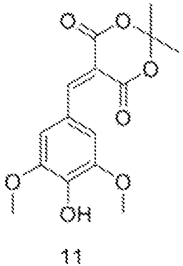 à savoir le 5-(4-hydroxy-3-methoxyphenyl)-2,2-dimethyl-1,3-dioxane-4,6-dione (composé 8), le 5-(3,4-dihydroxyphenyl)-2,2-dimethyl-1,3-dioxane-4,6-dione (composé 9), le 5-(4-Hydroxybenzylidene)-2,2-dimethyl-1,3-dioxane-4,6-dione (composé 10) et le 5-(4-hydroxy-3,5-dimethoxyphenyl)-2,2-dimethyl-1,3-dioxane-4,6-dione (composé 11).

Dans un mode de réalisation préféré, les composés utilisés pour leur propriétés anti-UV-A sont le composé 8 et le composé 11.

Ces dérivés phénoliques présentent également des propriétés anti-oxydantes (anti-radicalaires), qui s'ajoutent aux propriétés anti-UVA et anti-lumière bleue. Du fait de ces propriétés, ils trouvent leur application dans de nombreux domaines tels que la cosmétique, les polymères, l'agro-alimentaire, le phytosanitaire et la médecine.

En plus des propriétés de filtre anti-UVA et anti-lumière bleue, les composés de la série phénolique présentent des propriétés antipigmentantes et/ou dépigmentantes permettant leur utilisation en tant qu'agent éclaircissant pour la peau.

Dans un troisième mode de réalisation, le groupement R est de type pyrrole et répond à la formule (IV) 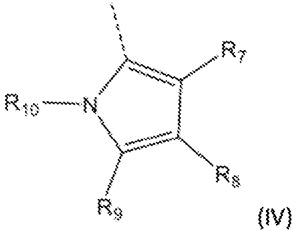 dans laquelle :
R₇ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle;
R₇ et R₈ peuvent être combinés pour former un cycle de type -(CH₂)ₙ- ou -(CH)ₙ- avec n allant de 4 à 8
R₈ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle
R₇ et R₈ peuvent être combinés pour former un cycle de type -(CH₂)ₙ- ou -(CH)ₙ- avec n allant de 4 à 8 R₈ et R₉ peuvent être combinés pour former un cycle de type -(CH₂)ₙ- ou -(CH)ₙ- avec n allant de 4 à 8
R₉ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle;
R₈ et R₉ peuvent être combinés pour former un cycle de type -(CH₂)ₙ- ou -(CH)ₙ- avec n allant de 4 à 8 R₉ et R₁₀ peuvent être combinés pour former un cycle de type -(CH₂)ₙ- ou -(CH)ₙ- avec n allant de 4 à 8
R₁₀ représente :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle.
R₉ et R₁₀ peuvent être combinés pour former un cycle de type -(CH₂)ₙ- ou -(CH)ₙ- avec n allant de 4 à 8.

Des exemples de dérivés pyrroliques de l'acide de Meldrum sont les composés 12 à 15 représentés ci-dessous : 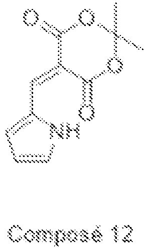 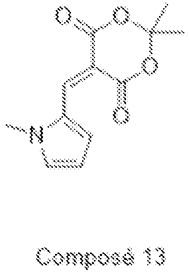 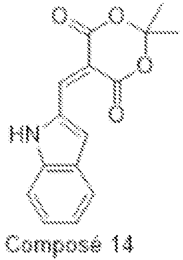 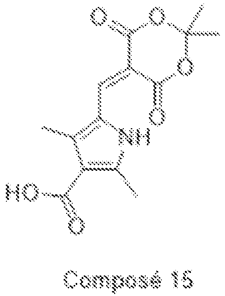 à savoir le 5-(1H-pyrrole-2-ylmethylidene)-2,2-dimethyl-1,3-dioxane-4,6-dione (composé 12), le 5-[(1-methyl)-pyrrole-2-ylmethylidene]-2,2-dimethyl-1,3-dioxane-4,6-dione (composé 13), le 5-[indol-2-ylmethylene]-2,2-dimethyl-1,3-dioxane-4,6-dione (composé 14) et le 5-[(1H-Pyrrole-4-carboxylic acid,-3,5-dimethyl)-2-ylmethylidene]-2,2-dimethyl-1,3-dioxane-4,6-dione (composé 15).

Dans un mode de réalisation préféré, les composés utilisés pour leur propriétés anti-UV-A sont le composé 12 et le composé 13.

Dans un quatrième mode de réalisation, le groupement R est de type vinylphénol et répond à la formule (V) 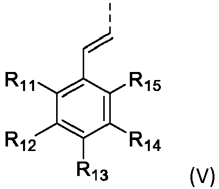 dans laquelle :
R₁₁, R₁₂, R₁₃, R₁₄, R₁₅ indépendamment choisis représentent :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle;
R₁₁ et R₁₂ peuvent être combinés pour former un cycle de type -(CH₂)ₙ- ou -(CH)ₙ- avec n allant de 4 à 8
R₁₂ et R₁₃ peuvent être combinés pour former un cycle de type -(CH₂)ₙ- ou -(CH)ₙ- avec n allant de 4 à 8
R₁₃ et R₁₄ peuvent être combinés pour former un cycle de type -(CH₂)ₙ- ou -(CH)ₙ- avec n allant de 4 à 8
R₁₄ et R₁₅ peuvent être combinés pour former un cycle de type -(CH₂)ₙ- ou -(CH)ₙ- avec n allant de 4 à 8

Des exemples de dérivés vinylphénol de l'acide de Meldrum sont les composés 16 à 18 représentés ci-dessous : 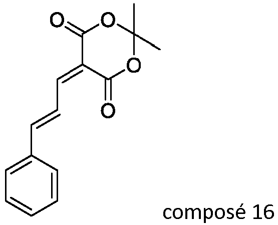 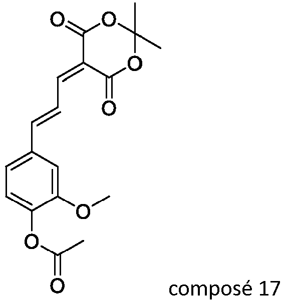 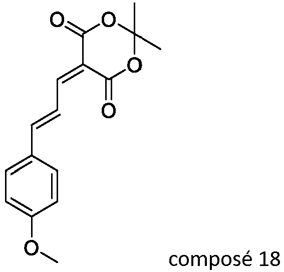 à savoir le 2,2-dimethyl-5-(3-phenyl-2-propenylidene)-1,3-Dioxane-4,6-dione (composé 16), le 2,2-dimethyl-5-(3-(4-acetoxy-3-methoxyphenyl)-2-propenylidene)-1,3-Dioxane-4,6-dione (composé 17) et le 2,2-dimethyl-5-(3-(4-methoxyphenyl)-2-propenylidene)-1,3-Dioxane-4,6-dione (composé 18)

Dans un mode de réalisation préféré, les composés utilisés pour leur propriétés anti-UV-A sont le composé 16 et le composé 17.

Dans un cinquième mode de réalisation, le groupement R est de type vinylfurane et répond à la formule (VI) 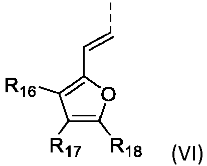 dans laquelle :
R₁₆, R₁₇, R₁₈, indépendamment choisis représentent :
   H, OH, CHO, CO₂H, NH₂, un groupement cyano, un halogène
   un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
   un groupement alkyle en C₁ à C₃₀ linéaire, cyclique ou branché, saturé ou insaturé
   une amine de type NHR, NR₂ ou NRR', avec R et R' indépendamment choisis parmi un groupement alkyle en C₁ à C₃₀ linéaire, cyclique ou branché, saturé ou insaturé
   un ester portant un groupement alkyle en C₁ à C₃₀ linéaire, cyclique ou branché, saturé ou insaturé, ou un groupement benzoyle
   un amide portant un groupement alkyle en C₁ à C₃₀ linéaire, cyclique ou branché, saturé ou insaturé
   une cétone portant un groupement alkyle en C₁ à C₃₀ linéaire, cyclique ou branché, saturé ou insaturé
   un éther portant un groupement alkyle en C₁ à C₃₀ linéaire, cyclique ou branché, saturé ou insaturé, ou un groupement silyle, époxyde ou benzyle;
R₁₆ et R₁₇ peuvent être combinés pour former un cycle de type -(CH₂)ₙ- ou -(CH)ₙ- avec n allant de 4 à 8 R₁₇ et R₁₃ peuvent être combinés pour former un cycle de type -(CH₂)ₙ- ou -(CH)ₙ- avec n allant de 4 à 8

Un exemple de dérivé vinylfurane de l'acide de Meldrum est le composé 19 représenté ci-dessous : 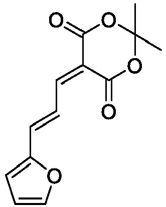 à savoir le 5-[3-(2-furanyl)-2-propen-1-ylidene]-2,2-dimethyl- 1,3-Dioxane-4,6-dione.

De manière générale, les dérivés de l'acide de Meldrum peuvent être utilisés seuls ou en mélange, permettant ainsi d'adapter les activités secondaires en fonction des spécificités de chaque type de dérivés, mais également de pouvoir couvrir à la fois les UVA et les UVB en combinant les dérivés de formule (I) avec des molécules présentant des propriétés anti-UVB.

Ainsi, dans un quatrième mode de réalisation de l'invention, différents composés de formule (I) sont utilisés en combinaison.

L'invention concerne alors l'utilisation d'au moins un dérivé de l'acide de Meldrum de formule (I), cette utilisation comprenant au moins deux dérivés d'acide de Meldrum choisis parmi (i) un dérivé substitué par un groupement de type furane, (ii) un dérivé substitué par un groupement de type phénol, (iii) un dérivé substitué par un groupement pyrrole, (iv) un dérivé substitué par un groupement de type vinylphénol et (v) un dérivé substitué par un groupement de type vinylfurane.

En d'autres termes, l'invention concerne par exemple :
- l'utilisation d'au moins deux dérivés de l'acide de Meldrum de formule (I) dans laquelle un dérivé de type furane est utilisé en combinaison avec un dérivé type phénol ;
- l'utilisation d'au moins deux dérivés de l'acide de Meldrum de formule (I) dans laquelle un dérivé de type furane est utilisé en combinaison avec un dérivé type pyrrole ;
- l'utilisation d'au moins deux dérivés de l'acide de Meldrum de formule (I) dans laquelle un dérivé de type furane est utilisé en combinaison avec un dérivé type vinylphénol ;
- l'utilisation d'au moins deux dérivés de l'acide de Meldrum de formule (I) dans laquelle un dérivé de type furane est utilisé en combinaison avec un dérivé type vinylfurane ;
- l'utilisation d'au moins deux dérivés de l'acide de Meldrum de formule (I) dans laquelle un dérivé de type phénol est utilisé en combinaison avec un dérivé type pyrrole ;
- l'utilisation d'au moins deux dérivés de l'acide de Meldrum de formule (I) dans laquelle un dérivé de type phénol est utilisé en combinaison avec un dérivé type vinylphénol ;
- l'utilisation d'au moins deux dérivés de l'acide de Meldrum de formule (I) dans laquelle un dérivé de type phénol est utilisé en combinaison avec un dérivé type vinylfurane ;
- l'utilisation d'au moins deux dérivés de l'acide de Meldrum de formule (I) dans laquelle un dérivé de type pyrrole est utilisé en combinaison avec un dérivé type vinylphénol ;
- l'utilisation d'au moins deux dérivés de l'acide de Meldrum de formule (I) dans laquelle un dérivé de type pyrrole est utilisé en combinaison avec un dérivé type vinylfurane ;
- l'utilisation d'au moins deux dérivés de l'acide de Meldrum de formule (I) dans laquelle un dérivé de type vinylphénol est utilisé en combinaison avec un dérivé type vinylfurane ;
- l'utilisation d'au moins deux dérivés furaniques de l'acide de Meldrum de formule (I) ;
- l'utilisation d'au moins deux dérivés phénoliques de l'acide de Meldrum de formule (I) ;
- l'utilisation d'au moins deux dérivés pyroliques de l'acide de Meldrum de formule (I).
- l'utilisation d'au moins deux dérivés vinylphénol de l'acide de Meldrum de formule (I).
- l'utilisation d'au moins deux dérivés vinylfurane de l'acide de Meldrum de formule (I).

Enfin, l'invention concerne aussi l'utilisation d'un dérivé de l'acide de Meldrum de formule (I) dans laquelle sont utilisés en combinaison au moins un dérivé de type furane, un dérivé de type phénol, un dérivé de type pyrrole, un dérivé de type vinylphénol ou un dérivé de type vinylfurane.

Pour élargir le spectre de protection aux UV-B, il est proposé d'utiliser les composés de formule (I) en combinaison avec des composés de type dimères conjugués présentant des propriétés anti-UV-B ou des dérivés du sinapoyl malate présentant également des propriétés anti UV-B. Les résultats expérimentaux ont permis de montrer la synergie de telles combinaisons et donc leur intérêt pour la protection anti-UV globale efficace.

Ainsi dans un sixième mode de réalisation, l'invention concerne l'utilisation d'un dérivé de l'acide de Meldrum de formule (I) tel que défini précédemment et comprend en outre au moins un dimère β-β de formule (VII) : 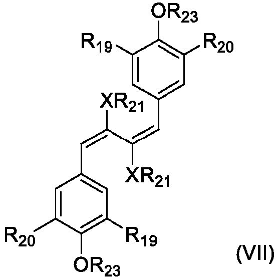 dans laquelle :
R₁₉ et R₂₀ sont indépendamment choisis parmi un groupement alkyle en C1 à C8 linéaire, cyclique ou branché, ou un groupement O-alkyle en C1 à C8 linéaire, cyclique ou branché ;
R₂₁ est un H, un groupement alkyle en C1 à C30 linéaire, cyclique ou branché ou un groupement hydro (mono- ou di-)acide organique ;
R₂₃ est un H, un groupement acétyle ou un groupement alkyle en C1 à C8 linéaire, cyclique ou branché, un groupe protecteur de type silyle, benzyle ou benzoyle ;
X est un groupement C=O, un groupement CO2 ou un groupement C(O)NR₂₂ dans lequel R₂₂ est un H ou un groupement alkyle en C1 à C8 linéaire, cyclique ou branché.

Dans un mode de réalisation préféré, le dimère β-β est choisi parmi le β-β disinapate d'éthyle, le β-β disinapate d'heptyle, le β-β disinapate de *tert*-butyle, le β-β disinapate d'*iso*-propyle le β-β disinapate de 2-éthylhexyle, le β-β disinapate de di*tert*-butyle malate, le β-β disinapate de crésol, le β-β disinapate de gaïacol, le β-β disinapate d'éthyle réduit, le β-β alcool disinapylique, le β-β disinapate de kojate, le β-β acétone disinapylique, le β-β disinapate de N-phénylamide et le β-β disinapate d'acide malique.

Des exemples de dimères β-β particulièrement préférés sont le dimère de β-β sinapate d'éthyle (β-β disinapate d'éthyle) dans lequel :
R₁₉ = R₂₀ = OMe,
X = CO₂,
R₂₁ = Et et
R₂₃ = H,
et le dimère β-β de sinapoyl malate (ou β-β disinapoyl malate) dans lequel :
R₁₉ = R₂₀ = OMe,
X = CO₂,
R₂₁ = CH(CO₂H)(CH₂CO₂H) et
R₂₃ = H.

Ces dimères β-β présentent des activités anti-UV, en particulier anti-UVB et/ou anti-oxydantes qui s'ajoutent à celles des dérivés de l'acide de Meldrum de formule (I). Des exemples d'activités mesurées pour de telle combinaisons sont présentés dans la partie expérimentale.

Dans un septième mode de réalisation, l'invention concerne l'utilisation d'un dérivé de l'acide de Meldrum de formule (I) tel que défini précédemment et comprend en outre au moins un dérivé du sinapoyl malate de formule (VIII) : 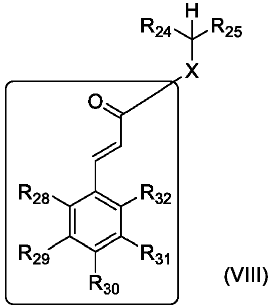 dans laquelle
R₂₄ est un groupement (CH₂)ₙCOOH ou (CH₂)ₙN(R₂₇)₃⁺X₂⁻
R₂₅ est un groupement H, (CH₂)ₙCOOH, (CH₂)ₙCH₃ , (CH₂)ₙ(CHOH)ₙCOOH, (CH₂)ₙ(CHOR₂₆)ₙCOOH, (CH₂)ₙN(R₂₇)₃⁺X₂⁻, (CH₂)ₙ(CHOH)ₙN(R₂₇)₃⁺X₂⁻ ou (CH₂)ₙ(CHOR₃)N(R₂₇)₃⁺X₂⁻
R₂₆ correspond au groupement A
R₂₇ est choisi parmi H ou un groupement CₙH₂ₙ₊₁
X₁ est un groupement O ou NH
X₂⁻ est un contre-ion négatif (anion) choisi parmi des halogénures, des acétates, des nitrates, des oxydes, des phosphates, sulfates, bisulfites, carboxylates, citrates
n étant un nombre entier compris entre 0 et 5
R₂₈, R₂₉, R₃₀, R₃₁ et R₃₂ sont indépendamment choisis parmi, un H, un OH, un NH₂, un SH, un halogène, un groupement alkyle en C₁ à C₈ linéaire, cyclique ou branché, saturé ou insaturé, un groupement O-alkyle en C₁ à C₈ linéaire, cyclique ou branché, saturé ou insaturé, un groupement NH-alkyle en C₁ à C₈ linéaire, cyclique ou branché, saturé ou insaturé ; un groupement N-(alkyle)₂ en C₁ à C₈ linéaire, cyclique ou branché, saturé ou insaturé.

Un huitième objet de l'invention concerne l'utilisation d'au moins un dérivé de l'acide de Meldrum de formule (I), seul ou en mélange, en tant que filtre UV-A et lumière bleue dans des compositions phytosanitaires, dans des compositions de protection contre le froid, dans des polymères ou dans des emballages.

Ces composés présentent aussi des propriétés de coloration dans les nuances rouge, jaune, orange et marron. Ils peuvent être utilisés pour colorer une formulation, par exemple une composition de maquillage (fond de teint, fard à paupière, rouge à lèvre), un additif polymère pour obtenir des plastiques colorés, packaging, ...

Dans un mode de réalisation préféré, les dérivés de l'acide de Meldrun sont choisis parmi les composés suivants : le 5-(2-Furanylmethylene)-2,2-dimethyl-1,3-dioxane-4,6-dione (composé 1), le 5-[(4,5-Dimethyl-2-furanyl)methylene]-2,2-dimethyl-1,3-dioxane-4,6-dione (composé 2), le 5-[(5-Ethyl-2-furanyl)methylene]-2,2-dimethyl-1,3-dioxane-4,6-dione (composé 3), le 5-[[5-(Hydroxymethyl)-2-furanyl]methylene]-2,2-dimethyl-1,3-dioxane-4,6-dione (composé 4), le 5-(4-hydroxy-3-methoxyphenyl)-2,2-dimethyl-1,3-dioxane-4,6-dione (composé 8), le 5-(4-hydroxy-3,5-dimethoxyphenyl)-2,2-dimethyl-1,3-dioxane-4,6-dione (composé 11), le 5-(1H-pyrrole-2-ylmethylidene)-2,2-dimethyl-1,3-dioxane-4,6-dione (composé 12), le 5-[(1-methyl)-pyrrole-2-ylmethylidene]-2,2-dimethyl-1,3-dioxane-4,6-dione (composé 13), le 2,2-dimethyl-5-(3-phenyl-2-propenylidene)-1,3-Dioxane-4,6-dione (composé 16), le 2,2-dimethyl-5-(3-(4-acetoxy-3-methoxyphenyl)-2-propenylidene)-1,3-Dioxane-4,6-dione (composé 17)et le 5-[3-(2-furanyl)-2-propen-1-ylidene]-2,2-dimethyl- 1,3-Dioxane-4,6-dione (composé 19).

La présente invention sera mieux comprise à la lecture des exemples qui suivent, fournis à titre d'illustration et ne devant en aucun cas être considérés comme limitant la portée de la présente invention.

### DESCRIPTION DES FIGURES

**Figure 1** **:** Procédé de préparation des dérivés d'acide de Meldrum
**Figure 2** **:** Spectres d'absorption aux UV des dérivés furaniques de l'acide de Meldrum (composés 1 à 7). Sont représentées : en abscisse les longueurs d'onde (en nm), et en ordonnée les absorbances.
**Figure 3** **:** Spectres d'absorption aux UV des composés 4 et 11 mesurés séparément ou en mélange. Sont représentées : en abscisse les longueurs d'onde (en nm), et en ordonnée les absorbances.
**Figure 4** **:** Spectres d'absorption aux UV du composé 1 et d'un dimère β-β mesurés séparément ou en mélange. Sont représentées : en abscisse les longueurs d'onde (en nm), et en ordonnée les absorbances.
**Figure** 5 : Spectres d'absorption aux UV des composés 1 et 2 et d'un dimère β-β mesurés séparément ou en mélange. Sont représentées : en abscisse les longueurs d'onde (en nm), et en ordonnée les absorbances.
**Figure 6** : Activités des récepteurs Erα, AR et PXR induites par la présence du composé 1 et du composé 4 à C = 10 µM.
**Figure 7** : Effet antagoniste du composé 1 et du composé 4 sur le récepteur Erα.
**Figure 8** **:** Effet antagoniste du composé 1 et du composé 4 sur le récepteur AR.
**Figure 9** **:** Effet antagoniste du composé 1 et du composé 4 sur le récepteur PXR.

### EXEMPLES

### EXEMPLE 1 : Synthèse des composés dérivés de l'acide de Meldrum

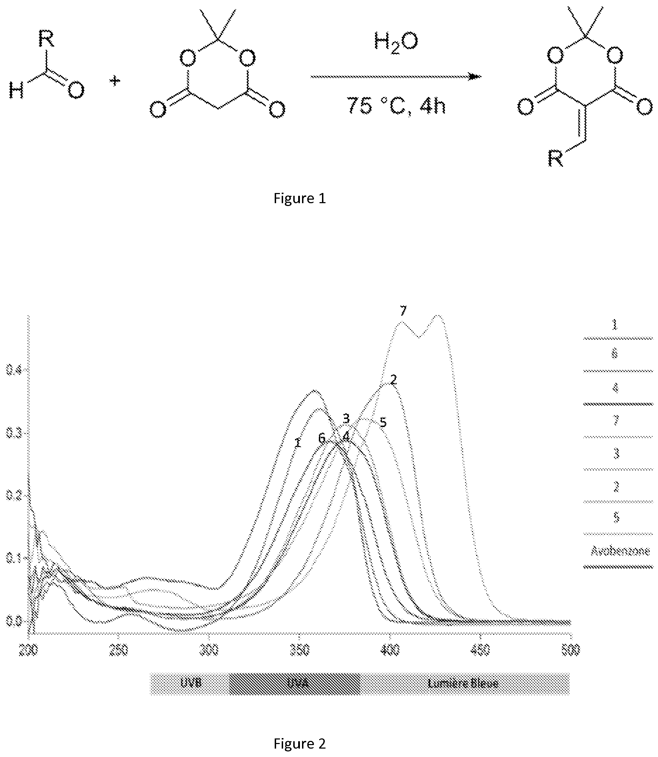

Les composés dérivés de l'acide de Meldrum de formule (I) sont obtenus par un procédé connu consistant à faire réagir l'acide de Meldrum avec un aldéhyde, tel que présenté à la Figure 1. Cette réaction s'effectue dans l'eau à 75 °C durant 4h.

Selon le type de dérivé synthétisé, le procédé met en jeu des aldéhydes furaniques, phénoliques (benzaldéhydes), pyrroliques, vinylfurane ou vinylphénol.

Dans la majorité des cas le produit précipite et est isolé par filtration. Si ce n'est pas le cas, une simple extraction liquide/liquide permet d'isoler le produit souhaité. Les rendements obtenus sont très bons (75-89%). Cette méthode permet d'accéder rapidement aux composés sans purification par chromatographie.

### EXEMPLE 2 : Propriétés anti-UVA et lumière bleue

Un spectre UV-Vis a été réalisé dans l'éthanol à 10⁻⁵ mol/L pour les composés 1 à 7, qui sont des dérivés furaniques de l'acide de Medrum. L'Avobenzone, une molécule très largement utilisée comme filtre UVA dans les crèmes solaires, a été utilisée en tant que référence.

Il apparait sur la Figure 2 que les composés 1 à 7 absorbent exactement au niveau des UVA (315-380 nm) ainsi qu'au niveau de la lumière bleue (380-500 nm).

Cette méthodologie a été employée pour évaluer les propriétés anti-UV des composés 8 à 11, qui sont des dérivés phénoliques de l'acide de Medrum, des composés 12 à 15 qui sont des dérivés pyrroliques de l'acide de Meldrum, des composés 16 à 18 qui sont des dérivés vinylphénol de l'acide de Meldrum, le composé 19 qui est un dérivé vinylfurane de l'acide de Meldrum. Les résultats obtenus sont comparables à ceux obtenus avec les composés 1 à 7.

### EXEMPLE 3 : Photostabilité des dérivés de l'acide de Meldrum

La stabilité des composés 1 à 7 aux UV a été étudiée par soumission à une exposition (λ : 300 nm, P : 8.32 W/m²) pendant une heure.

L'Avobenzone a été utilisée comme molécule de référence. Cette molécule est connue pour être extrêmement stable sous exposition UV, ce qui n'est pas du tout le cas d'autres composés bio-sourcés.

Les résultats du Tableau 1 montrent que certains dérivés furaniques de l'acide de Meldrum présentent une stabilité comparable à l'Avobenzone (perte d'activité < à 5%), en particulier les composés 1, 4, 2 et 3.

**Tableau 1 : Stabilité des composés 1 à 7 soumis à une exposition aux UV.**

| **Molecules** | **λₘₐₓ** | **A (t=0 min)** | **Perte d'activité (%)** |
|---|---|---|---|
| Avobenzone | 350 | 0,36735 | 0,6 |
| Composé **(1)** | 361 | 0.33809 | 3.5 |
| Composé **(4)** | 374 | 0,28814 | 3,4 |
| Composé **(6)** | 366 | 0,28728 | 12,0 |
| Composé **(5)** | 386 | 0,32310 | 28,7 |
| Composé **(7)** | 406 - 426 | 0,47642 | 12,4 |
| Composé **(3)** | 374 | 0,31307 | 5,1 |
| Composé **(2)** | 400 | 0,37961 | 4,2 |

La photostabilité des composés 8 à 11 a également été étudiée, de manière identique au test appliqué aux composés 1 à 7. Les résultats obtenus sont présentés dans le Tableau 2. Ils montrent des stabilités comparables à ceux obtenus avec les composés 1 à 7. Cette méthodologie a été appliquée à l'ensemble des molécules décrites ci-dessus.

**Tableau 2 : Stabilité des composés 8 à 11 soumis à une exposition aux UV.**

| **Molécules** | **λₘₐₓ** | **A (t=0 min)** | **Perte d'activité (%)** |
|---|---|---|---|
| Composé 8 | 393 | 0,29153 | 9,00 |
| Composé 9 | 398 | 0,26515 | 6,37 |
| Composé 10 | 374 | 0,29431 | 3,47 |
| Composé 11 | 405 | 0,24614 | 11,43 |

### EXEMPLE 4 : Propriétés anti-pigmentantes (non couvertes par les présentes revendications)

Les propriétés anti-pigmentantes des composés 8 à 11 ont été évaluées en analysant leur capacité à inhiber la tyrosinase.

L'Acide Kojic a été utilisé comme référence en termes d'inhibition de la tyrosinase. Ainsi, les IC₅₀ des composés 8 à 11 ont été comparées avec celle de l'Acide Kojic.

Les résultats sont présentés dans le Tableau 3 et montrent des activités intéressantes.

| **Molécules** | **IC₅₀ (µM)** |
|---|---|
| **Acide Kojic** | **18** |
| Composé **8** | **23** |
| Composé **9** | **13** |
| Composé **10** | 106 |
| Composé **11** | **17** |

| | |
|---|---|
| **Tableau 3** : Capacité d'inhibition de la tyrosinase des dérivés phénoliques de l'acide de Meldrum | |

### EXEMPLE 5 : Propriétés anti-oxydantes (non couvertes par les présentes revendications)

Les propriétés anti-oxydantes des composés 8 à 11 ont été évaluées par comparaison de leur IC₅₀ avec celles de deux molécules de référence que sont la BHA et le BHT. Ces deux molécules sont très largement utilisées dans les formulations cosmétiques bien qu'elles soient controversées pour leur effet potentiellement cancérigène.

Les résultats sont présentés au Tableau 4.

**Tableau 4 : Propriétés anti-oxydantes des dérivés phénoliques de l'acide de Meldrum**

| **Molécules** | **IC₅₀ (nmol)** |
|---|---|
| **BHA** | **6,51** |
| **BHT** | **13,13** |
| Composé **8** | 53,77 |
| Composé **9** | **2,90** |
| Composé **10** | Pas d'effet |
| Composé **11** | **3,58** |

Les résultats obtenus sont très comparables à ceux des deux références utilisées. En particulier, la capacité antioxydante des composés 9 et 11 sont remarquables. Ces résultats confirment les données de la littérature puisque le pouvoir antioxydant des composés 8 à 11 a déjà fait l'objet de publications scientifiques.

### EXEMPLE 6 : Evaluation de l'effet des dérivés de l'acide de Meldrum sur le système endocrinien (non couvertes par les présentes revendications)

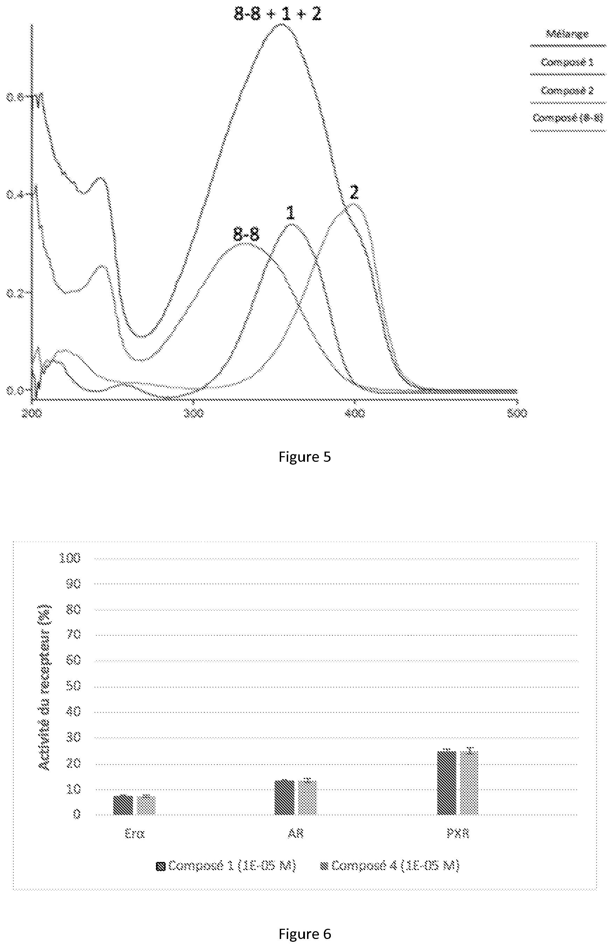

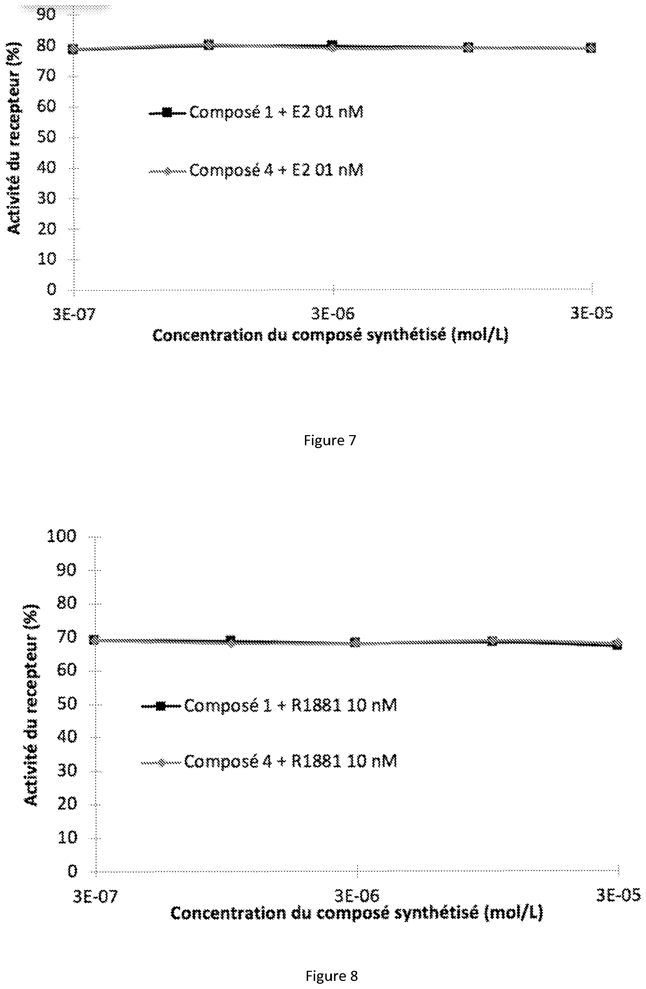

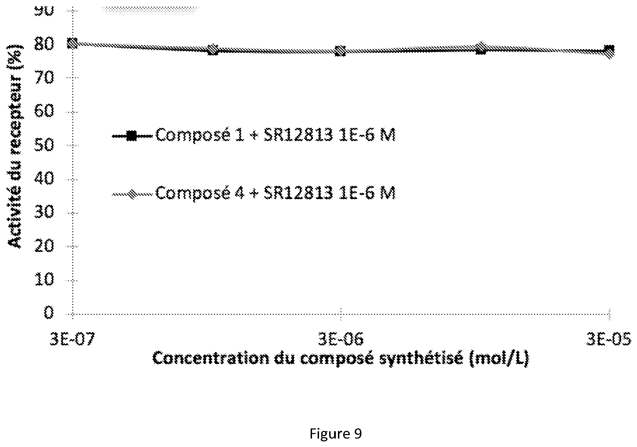

L'effet des composés 1 et 4 sur le système endocrinien a été évalué pour leurs propriétés agonistes et antagonistes sur différents récepteurs : ERα (récepteur œstrogène alpha), AR (récepteur des androgènes) et PXR (pregnane X receptor). Un premier test consiste à étudier l'activité des récepteurs en présence du composé 1 ou 4 (effet agoniste). Les résultats présentés dans la figure 6 montrent que les composés synthétisés n'induisent pas une activité anormale des récepteurs même aux concentrations les plus élevées. Un second test vise à l'étude de l'activité des récepteurs en présence du composé 1 ou 4 et d'une molécule de référence pour chaque récepteur (effet antagoniste). Les résultats mis en évidence dans les figures 7 à 9 montrent que les composés synthétisés n'empêchent pas l'activité des récepteurs envers les molécules de référence, même aux concentrations les plus élevées.

Les résultats obtenus mettent en avant une absence d'effet agoniste et antagoniste des composés 1 et 4 sur les récepteurs étudiés, ces molécules ne possédant pas d'effet négatifs sur le système endocrinien.

### EXEMPLE 7 : Propriétés d'un mélange de différents dérivés de l'acide de Meldrum

Les différents dérivés de l'acide de Meldrum peuvent être utilisés en mélange. Ceci permet de préparer des compositions dans lesquelles les propriétés anti-UVA des composés se cumulent mais aussi de conférer des propriétés additionnelles à la composition anti-UV en fonction des propriétés secondaires des composés mélangés.

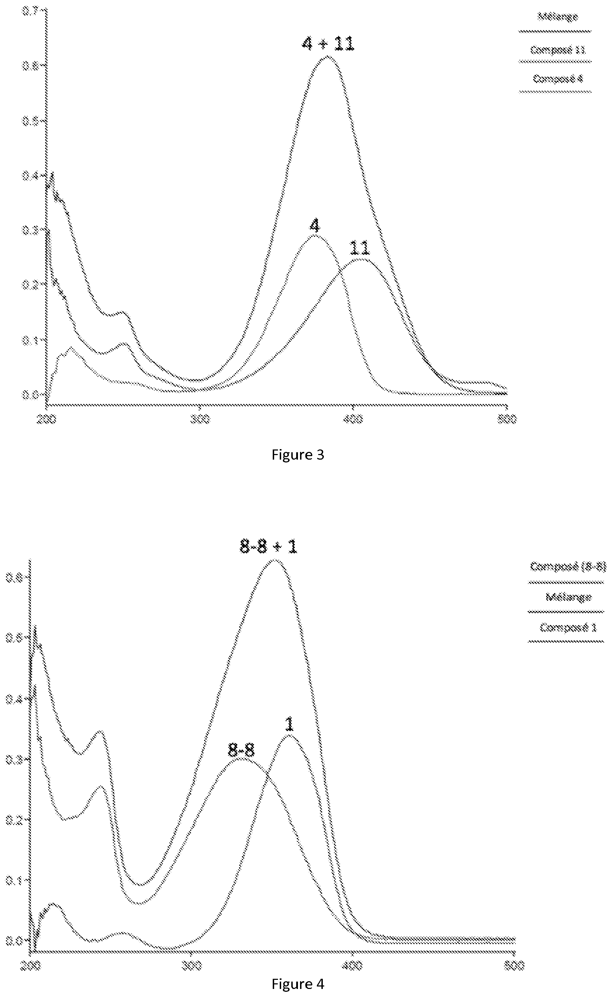

Ainsi, la combinaison d'un composé du groupe furanique (composé 4) avec un composé du groupe phénolique (composé 11) permet d'augmenter la gamme de protection. Le résultat est présenté à la Figure 3 et montre une augmentation de l'absorbance et une couverture plus intense sur la gamme 250-400 nm.

### EXEMPLE 8 : Propriétés d'un mélange de dérivés de l'acide de Meldrum et de dimère β-β

L'utilisation d'un dérivé de l'acide de Meldrum de formule (I) en tant que filtre UVA et d'un dimère β-β de formule (V) en tant que filtre UVB permet clairement d'augmenter la gamme de protection. La présence d'un dimère β-β confère de plus une activité antioxydante au mélange.

Une première combinaison a consisté à mélanger un dérivé furanique de l'acide de Meldrum (composé 1) et un dimère β-β (β-β disinapate d'isopropyle). Pris individuellement, à concentrations égales, ces deux composés absorbent sur des gammes de longueurs d'ondes différentes. La combinaison des deux permet d'augmenter la gamme d'absorbance et de couvrir de manière plus intense de 250 à 400 nm (UVA et UVB). Les résultats d'une telle combinaison sont présentés à la Figure 4.

On s'attend à ce que ce mélange présente également un pouvoir anti-oxydant du fait de la présence du dimère.

Une deuxième combinaison à trois composés a également été testée, à savoir deux dérivés furaniques de l'acide de Meldrum (composés 1 et 2) et un dimère β-β (β-β disinapate d'isopropyl). Le résultat est présenté à la Figure 5.

L'absorbance est de fait supérieure et la gamme recouvre celles des trois composés pris séparément avec une intensité nettement supérieure. La présence d'un composé de la série phénolique ou d'un composé dimère β-β dans ce mélange implique une activité anti-oxydante du mélange. On s'attend à ce que la présence d'un composé de la série phénolique confère une activité anti-tyrosinase au mélange.

## Revendications

1. Dérivé de l'acide de Meldrum de formule (I) 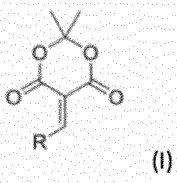
dans laquelle R est un groupement de type furane, de type phénol, de type pyrrole, de type vinylphénol ou de type vinylfurane,
pour son utilisation pour la peau en tant que filtre UV-A et lumière bleue.

2. Utilisation d'au moins d'un dérivé de l'acide de Meldrum de formule (I) 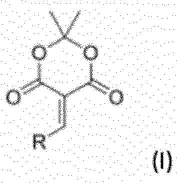
dans laquelle R est un groupement de type furane, de type phénol, de type pyrrole, de type vinylphénol ou de type vinylfurane,
en tant que filtre UV-A et lumière bleue dans des compositions phytosanitaires, dans des compositions de protection contre le froid, dans des polymères ou dans des emballages.

3. Dérivé selon la revendication 1 pour son utilisation selon la revendication 1 ou utilisation selon la revendication 2, dans lequel/laquelle ledit groupement R de type furane répond à la formule (II) 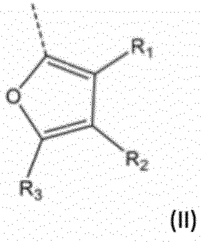 dans laquelle :
R₁ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₁ et R₂ peuvent être combinés pour former un cycle de type -(CH₂)ₙ- ou -(CH)ₙ- avec n allant de 4 à 8
R₂ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₁ et R₂ peuvent être combinés pour former un cycle de type -(CH₂)ₙ- ou -(CH)ₙ- avec n allant de 4 à 8
R₂ et R₃ peuvent être combinés pour former un cycle de type -(CH₂)ₙ- ou -(CH)ₙ- avec n allant de 4 à 8
R₃ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle
R₂ et R₃ peuvent être combinés pour former un cycle de type -(CH₂)ₙ- ou -(CH)ₙ- avec n allant de 4 à 8.

4. Dérivé selon la revendication 1 pour son utilisation selon la revendication 1 ou utilisation selon la revendication 2, dans lequel/laquelle ledit groupement R de type phénol répond à la formule (III) 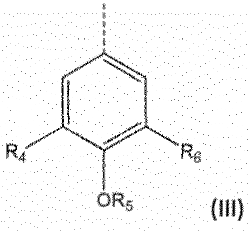 dans laquelle :
R₄ et R₆ sont indépendamment choisis parmi, un H, ou un groupement alkyle en C₁ à C₈ linéaire, cyclique ou branché, saturé ou insaturé, ou un groupement O-alkyle en C₁ à C₈ linéaire, cyclique ou branché, saturé ou insaturé
R₅ est un H, un groupement acétyle ou un groupement alkyle en C₁ à C₈ linéaire, cyclique ou branché, saturé ou insaturé, un groupe protecteur de type silyle, époxyde, benzyle ou benzoyle.

5. Dérivé selon la revendication 1 pour son utilisation selon la revendication 1 ou utilisation selon la revendication 2, dans lequle/laquelle ledit groupement R de type pyrrole répond à la formule (IV) 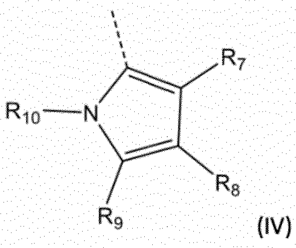 dans laquelle :
R₇ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₇ et R₈ peuvent être combinés pour former un cycle de type -(CH₂)ₙ- ou -(CH)ₙ- avec n allant de 4 à 8
R₈ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₇ et R₈ peuvent être combinés pour former un cycle de type -(CH₂)ₙ- ou -(CH)ₙ- avec n allant de 4 à 8
R₈ et R₉ peuvent être combinés pour former un cycle de type -(CH₂)ₙ- ou -(CH)ₙ- avec n allant de 4 à 8
R₉ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₈ et R₉ peuvent être combinés pour former un cycle de type -(CH₂)ₙ- ou -(CH)ₙ- avec n allant de 4 à 8
R₉ et R₁₀ peuvent être combinés pour former un cycle de type -(CH₂)ₙ- ou -(CH)ₙ- avec n allant de 4 à 8
R₁₀ représente :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle
R₉ et R₁₀ peuvent être combinés pour former un cycle de type -(CH₂)ₙ- ou -(CH)ₙ- avec n allant de 4 à 8.

6. Dérivé selon la revendication 1 pour son utilisation selon la revendication 1 ou utilisation selon la revendication 2, dans lequel/laquelle ledit groupement R de type vinylphénol répond à la formule (V) 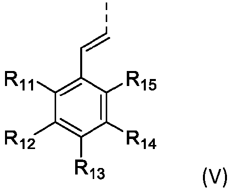 dans laquelle :
R₁₁, R₁₂, R₁₃, R₁₄, R₁₅ indépendamment choisis représentent :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₁₁ et R₁₂ peuvent être combinés pour former un cycle de type -(CH₂)ₙ- ou -(CH)ₙ- avec n allant de 4 à 8
R₁₂ et R₁₃ peuvent être combinés pour former un cycle de type -(CH₂)ₙ- ou -(CH)ₙ- avec n allant de 4 à 8
R₁₃ et R₁₄ peuvent être combinés pour former un cycle de type -(CH₂)ₙ- ou -(CH)ₙ- avec n allant de 4 à 8
R₁₄ et R₁₅ peuvent être combinés pour former un cycle de type -(CH₂)ₙ- ou -(CH)ₙ- avec n allant de 4 à 8

7. Dérivé selon la revendication 1 pour son utilisation selon la revendication 1 ou utilisation selon la revendication 2 dans lequel/laquelle ledit groupement R de type vinylfurane répond à la formule (VI) 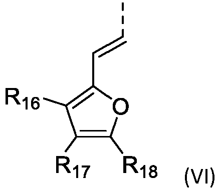 dans laquelle :
R₁₆, R₁₇, R₁₈, indépendamment choisis représentent :
H, OH, CHO, CO₂H, NH₂, un groupement cyano, un halogène
un groupement (CH₂)ₓOH ou (CH₂)ₓNH₂ ou (CH₂)ₓCO, ou (CH₂)ₓCOOH linéaire, cyclique ou branché, saturé ou insaturé, avec x allant de 1 à 30
un groupement alkyle en C₁ à C₃₀ linéaire, cyclique ou branché, saturé ou insaturé
une amine de type NHR, NR₂ ou NRR', avec R et R' indépendamment choisis parmi un groupement alkyle en C₁ à C₃₀ linéaire, cyclique ou branché, saturé ou insaturé
un ester portant un groupement alkyle en C₁ à C₃₀ linéaire, cyclique ou branché, saturé ou insaturé, ou un groupement benzoyle
un amide portant un groupement alkyle en C₁ à C₃₀ linéaire, cyclique ou branché, saturé ou insaturé
une cétone portant un groupement alkyle en C₁ à C₃₀ linéaire, cyclique ou branché, saturé ou insaturé
un éther portant un groupement alkyle en C₁ à C₃₀ linéaire, cyclique ou branché, saturé ou insaturé, ou un groupement silyle, époxyde ou benzyle;
R₁₆ et R₁₇ peuvent être combinés pour former un cycle de type -(CH₂)ₙ- ou -(CH)ₙ- avec n allant de 4 à 8
R₁₇ et R₁₃ peuvent être combinés pour former un cycle de type -(CH₂)ₙ- ou -(CH)ₙ- avec n allant de 4 à 8.

8. Dérivé selon l'une des revendications 1 à 7 pour son utilisation selon l'une des revendications 1 à 7 ou utilisation selon l'une des revendications 1 à 7 comprenant au moins deux dérivés d'acide de Meldrum tels que définis à la revendication 1 ou 2 choisis parmi (i) un dérivé substitué par un groupement de type furane, (ii) un dérivé substitué par un groupement de type phénol et (iii) un dérivé substitué par un groupement pyrrole, (iv) un dérivé substitué par un groupement vinylphénol et (v) un dérivé substitué par un groupement vinylfurane.

9. Dérivé selon l'une des revendications précédentes pour son utilisation selon l'une des revendications précédentes ou utilisation selon l'une des revendications précédentes comprenant en outre au moins un dimère β-β de formule (VII) : 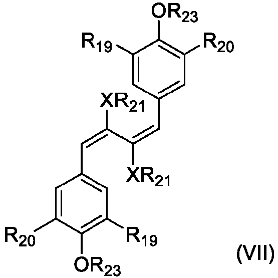 dans laquelle :
R₁₉ et R₂₀ sont indépendamment choisis parmi un groupement alkyle en C1 à C8 linéaire, cyclique ou branché, ou un groupement O-alkyle en C1 à C8 linéaire, cyclique ou branché ;
R₂₁ est un H, un groupement alkyle en C1 à C30 linéaire, cyclique ou branché ou un groupement hydro (mono- ou di-)acide organique ;
R₂₃ est un H, un groupement acétyle ou un groupement alkyle en C1 à C8 linéaire, cyclique ou branché, un groupe protecteur de type silyle, benzyle ou benzoyle ;
X est un groupement C=O, un groupement CO2 ou un groupement C(O)NR₂₂ dans lequel R₂₂ est un H ou un groupement alkyle en C1 à C8 linéaire, cyclique ou branché.

10. Dérivé selon l'une des revendications précédentes pour son utilisation selon l'une des revendications précédentes ou utilisation selon l'une des revendications précédentes comprenant en outre au moins un dérivé du sinapoyl malate de formule (VIII) : 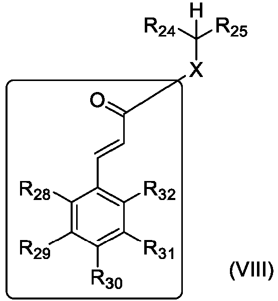 dans laquelle
R₂₄ est un groupement (CH₂)ₙCOOH ou (CH₂)ₙN(R₂₇)₃⁺X₂⁻
R₂₅ est un groupement H, (CH₂)ₙCOOH, (CH₂)ₙCH₃ , (CH₂)ₙ(CHOH)ₙCOOH, (CH₂)ₙ(CHOR₂₆)ₙCOOH, (CH₂)ₙN(R₂₇)₃⁺X₂⁻, (CH₂)ₙ(CHOH)ₙN(R₂₇)₃⁺X₂⁻ ou (CH₂)ₙ(CHOR₃)N(R₂₇)₃⁺X₂⁻
R₂₆ correspond au groupement A
R₂₇ est choisi parmi H ou un groupement CₙH₂ₙ₊₁
X₁ est un groupement O ou NH
X₂⁻ est un contre-ion négatif (anion) choisi parmi des halogénures, des acétates, des nitrates, des oxydes, des phosphates, sulfates, bisulfites, carboxylates, citrates
n étant un nombre entier compris entre 0 et 5
R₂₈, R₂₉, R₃₀, R₃₁ et R₃₂ sont indépendamment choisis parmi, un H, un OH, un NH₂, un SH, un halogène, un groupement alkyle en C₁ à C₈ linéaire, cyclique ou branché, saturé ou insaturé, un groupement O-alkyle en C₁ à C₈ linéaire, cyclique ou branché, saturé ou insaturé, un groupement NH-alkyle en C₁ à C₈ linéaire, cyclique ou branché, saturé ou insaturé ; un groupement N-(alkyle)₂ en C₁ à C₈ linéaire, cyclique ou branché, saturé ou insaturé.

11. Dérivé selon l'une des revendications précédentes pour son utilisation selon l'une des revendications précédentes ou utilisation selon l'une des revendications précédentes dans lequel/laquelle ledit dérivé de l'acide de Meldrum est parmi les composés suivants : le 5-(2-Furanylmethylene)-2,2-dimethyl-1,3-dioxane-4,6-dione (composé 1), le 5-[(4,5-Dimethyl-2-furanyl)methylene]-2,2-dimethyl-1,3-dioxane-4,6-dione (composé 2), le 5-[(5-Ethyl-2-furanyl)methylene]-2,2-dimethyl-1,3-dioxane-4,6-dione (composé 3), le 5-[[5-(Hydroxymethyl)-2-furanyl]methylene]-2,2-dimethyl-1,3-dioxane-4,6-dione (composé 4), le 5-(4-hydroxy-3-methoxyphenyl)-2,2-dimethyl-1,3-dioxane-4,6-dione (composé 8), le 5-(4-hydroxy-3,5-dimethoxyphenyl)-2,2-dimethyl-1,3-dioxane-4,6-dione (composé 11), le 5-(1H-pyrrole-2-ylmethylidene)-2,2-dimethyl-1,3-dioxane-4,6-dione (composé 12), le 5-[(1-methyl)-pyrrole-2-ylmethylidene]-2,2-dimethyl-1,3-dioxane-4,6-dione (composé 13), le 2,2-dimethyl-5-(3-phenyl-2-propenylidene)-1,3-Dioxane-4,6-dione (composé 16), le 2,2-dimethyl-5-(3-(4-acetoxy-3-methoxyphenyl)-2-propenylidene)-1,3-Dioxane-4,6-dione (composé 17)et le 5-[3-(2-furanyl)-2-propen-1-ylidene]-2,2-dimethyl- 1,3-Dioxane-4,6-dione (composé 19).

## Patentansprüche

1. Derivat der Meldrumsäure der Formel (I), 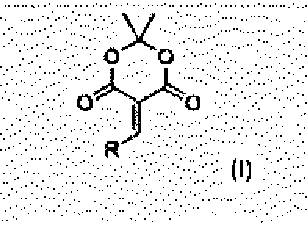
wobei R eine Gruppe vom Furan-Typ, vom Phenol-Typ, vom Pyrrol-Typ, vom Vinylphenol-Typ oder vom Vinylfuran-Typ ist,
zur Verwendung auf der Haut als UV-A- und Blaulichtfilter.

2. Verwendung von mindestens einem Derivat der Meldrumsäure der Formel (I), 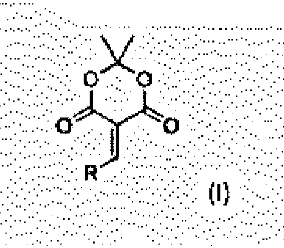
wobei R eine Gruppe vom Furan-Typ, vom Phenol-Typ, vom Pyrrol-Typ, vom Vinylphenol-Typ oder vom Vinylfuran-Typ ist,
als UV-A- und Blaulichtfilter in Pflanzenschutzmittelzusammensetzungen, in Kälteschutzzusammensetzungen, in Polymeren oder in Verpackungen.

3. Derivat nach Anspruch 1 zur Verwendung dieses Derivats nach Anspruch 1 oder zur Verwendung nach Anspruch 2, wobei die Gruppe R vom Furan-Typ der Formel (II) entspricht 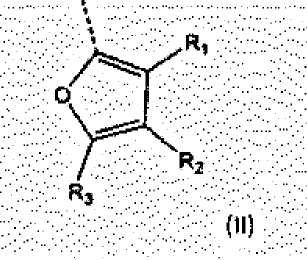 wobei:
R₁ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
wobei R₁ und R₂ kombiniert werden können, um einen Ring vom Typ - (CH₂)ₙ- oder -(CH)ₙ- auszubilden, wobei n von 4 bis 8 reicht
R₂ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
wobei R₁ und R₂ kombiniert werden können, um einen Ring vom Typ - (CH₂)ₙ- oder -(CH)ₙ- auszubilden, wobei n von 4 bis 8 reicht
R₂ und R₃ kombiniert werden können, um einen Ring vom Typ -(CH₂)ₙ- oder -(CH)ₙ- auszubilden, wobei n von 4 bis 8 reicht
R₃ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt
R₂ und R₃ kombiniert werden können, um einen Ring vom Typ -(CH₂)ₙ- oder -(CH)ₙ- auszubilden, wobei n von 4 bis 8 reicht.

4. Derivat nach Anspruch 1 zur Verwendung dieses nach Anspruch 1 oder zur Verwendung nach Anspruch 2, wobei die Gruppe R vom Phenol-Typ der Formel (III) entspricht 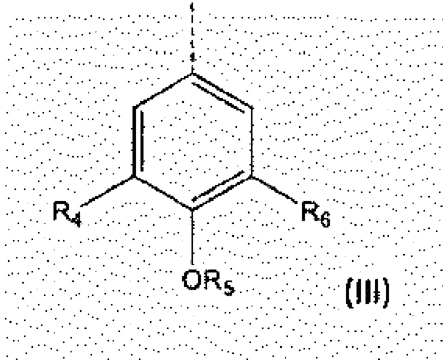 wobei:
R₄ und R₆ unabhängig ausgewählt sind aus H oder einer linearen, cyclischen oder verzweigten, gesättigten oder ungesättigten C₁- bis C₈-AlkylGruppe oder einer linearen, cyclischen oder verzweigten, gesättigten oder ungesättigten C₁- bis C₈-O-Alkyl-Gruppe
R₅ ein H, eine Acetyl-Gruppe oder eine lineare, cyclische oder verzweigte, gesättigte oder ungesättigte C₁- bis C₈-Alkyl-Gruppe, eine Schutzgruppe vom Silyl-, Epoxid-, Benzyl- oder Benzoyl-Typ ist.

5. Derivat nach Anspruch 1 zur Verwendung dieses nach Anspruch 1 oder zur Verwendung nach Anspruch 2, wobei die Gruppe R vom Pyrrol-Typ der Formel (IV) entspricht 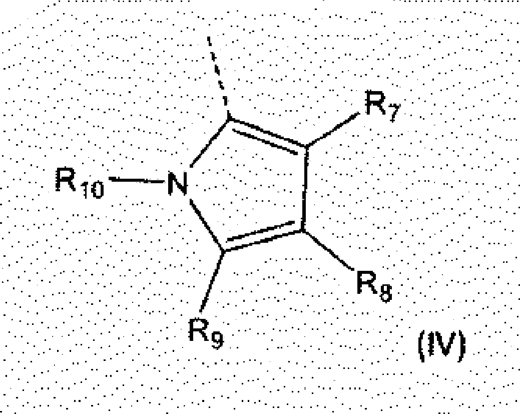 wobei:
R₇ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
R₇ und R₈ kombiniert werden können, um einen Ring vom Typ -(CH₂)ₙ- oder -(CH)ₙ- auszubilden, wobei n von 4 bis 8 reicht
R₈ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
R₇ und R₈ kombiniert werden können, um einen Ring vom Typ -(CH₂)ₙ- oder -(CH)ₙ- auszubilden, wobei n von 4 bis 8 reicht
R₈ und R₉ kombiniert werden können, um einen Ring vom Typ -(CH₂)ₙ- oder -(CH)ₙ- auszubilden, wobei n von 4 bis 8 reicht
R₉ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
R₈ und R₉ kombiniert werden können, um einen Ring vom Typ -(CH₂)ₙ- oder -(CH)ₙ- auszubilden, wobei n von 4 bis 8 reicht
R₉ und R₁₀ kombiniert werden können, um einen Ring vom Typ -(CH₂)ₙ- oder -(CH)ₙ- auszubilden, wobei n von 4 bis 8 reicht
R₁₀ darstellt:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt
R₉ und R₁₀ kombiniert werden können, um einen Ring vom Typ -(CH₂)ₙ- oder -(CH)ₙ- auszubilden, wobei n von 4 bis 8 reicht.

6. Derivat nach Anspruch 1 zur Verwendung dieses nach Anspruch 1 oder zur Verwendung nach Anspruch 2, wobei die Gruppe R vom Vinylphenol-Typ der Formel (V) entspricht 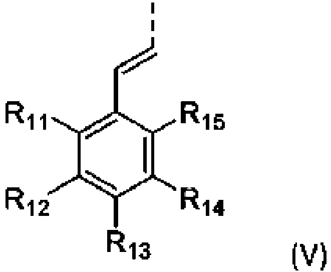 wobei:
R₁₁, R₁₂, R₁₃, R₁₄, R₁₅ unabhängig voneinander ausgewählt sind aus:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁-bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
R₁₁ und R₁₂ kombiniert werden können, um einen Ring vom Typ -(CH₂)ₙ- oder -(CH)ₙ- auszubilden, wobei n von 4 bis 8 reicht
R₁₂ und R₁₃ kombiniert werden können, um einen Ring vom Typ -(CH₂)ₙ- oder -(CH)ₙ- auszubilden, wobei n von 4 bis 8 reicht
R₁₃ und R₁₄ kombiniert werden können, um einen Ring vom Typ -(CH₂)ₙ- oder -(CH)ₙ- auszubilden, wobei n von 4 bis 8 reicht
R₁₄ und R₁₅ kombiniert werden können, um einen Ring vom Typ -(CH₂)ₙ- oder -(CH)ₙ- auszubilden, wobei n von 4 bis 8 reicht

7. Derivat nach Anspruch 1 zur Verwendung dieses nach Anspruch 1 oder zur Verwendung nach Anspruch 2, wobei die Gruppe R vom Vinylfuran-Typ der Formel (VI) entspricht 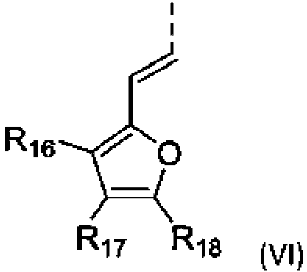 wobei:
R₁₆, R₁₇, R₁₈, unabhängig voneinander ausgewählt sind aus:
H, OH, CHO, CO₂H, NH₂, eine Cyano-Gruppe, ein Halogen
eine Gruppe (CH₂)ₓOH oder (CH₂)ₓNH₂ oder (CH₂)ₓCO, oder (CH₂)ₓCOOH linear, cyclisch oder verzweigt, gesättigt oder ungesättigt, wobei x von 1 bis 30 reicht
eine lineare, cyclische oder verzweigte, gesättigte oder ungesättigte C₁- bis C₃₀-Alkyl-Gruppe
ein Amin vom Typ NHR, NR₂ oder NRR', wobei R und R' unabhängig ausgewählt sind aus einer linearen, cyclischen oder verzweigten, gesättigten oder ungesättigten C₁- bis C₃₀-Alkyl-Gruppe
ein Ester, der eine lineare, cyclische oder verzweigte, gesättigte oder ungesättigte C₁- bis C₃₀-Alkyl-Gruppe oder eine Benzoyl-Gruppe trägt
ein Amid, das eine lineare, cyclische oder verzweigte, gesättigte oder ungesättigte C₁- bis C₃₀-Alkyl-Gruppe trägt
ein Keton, das eine lineare, cyclische oder verzweigte, gesättigte oder ungesättigte C₁- bis C₃₀-Alkyl-Gruppe trägt
ein Ether, der eine lineare, cyclische oder verzweigte, gesättigte oder ungesättigte C₁- bis C₃₀-Alkyl-Gruppe oder eine Silyl-, Epoxid- oder BenzylGruppe trägt;
R₁₆ und R₁₇ kombiniert werden können, um einen Ring vom Typ -(CH₂)ₙ- oder -(CH)ₙ- auszubilden, wobei n von 4 bis 8 reicht
R₁₇ und R₁₈ kombiniert werden können, um einen Ring vom Typ -(CH₂)ₙ- oder -(CH)ₙ- auszubilden, wobei n von 4 bis 8 reicht.

8. Derivat nach einem der Ansprüche 1 bis 7 zur Verwendung dieses nach einem der Ansprüche 1 bis 7 oder zur Verwendung nach einem der Ansprüche 1 bis 7, umfassend mindestens zwei Meldrumsäure-Derivate wie in Anspruch 1 oder 2 definiert, ausgewählt aus (i) einem durch eine Furan-Gruppe substituierten Derivat, (ii) einem durch eine Phenol-Gruppe substituierten Derivat und (iii) einem durch eine Pyrrol-Gruppe substituierten Derivat, (iv) einem durch eine Vinylphenol-Gruppe substituierten Derivat und (v) einem durch eine Vinylfuran-Gruppe substituierten Derivat.

9. Derivat nach einem der vorstehenden Ansprüche zur Verwendung dieses nach einem der vorstehenden Ansprüche oder zur Verwendung nach einem der vorstehenden Ansprüche, ferner umfassend mindestens ein β-β-Dimer der Formel (VII): 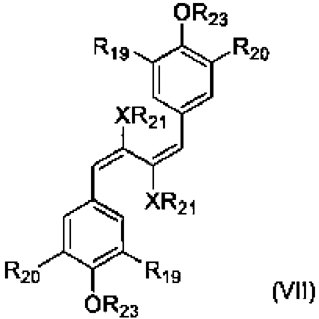 wobei:
R₁₉ und R₂₀ unabhängig ausgewählt sind aus einer linearen, cyclischen oder verzweigten C1- bis C8-Alkyl-Gruppe oder einer linearen, cyclischen oder verzweigten C1- bis C8-O-Alkyl-Gruppe;
R₂₁ ein H, eine lineare, cyclische oder verzweigte C1- bis C30-AlkylGruppe oder eine organische Hydro(mono- oder di-)säuregruppe ist;
R₂₃ ein H, eine Acetylgruppe oder eine lineare, cyclische oder verzweigte C1- bis C8-Alkyl-Gruppe, eine Schutzgruppe vom Silyl-, Benzyl- oder Benzoyl-Typ ist;
X eine C=O-Gruppe, eine CO2-Gruppe oder eine C(O)NR₂₂-Gruppe ist, wobei R₂₂ ein H oder eine lineare, cyclische oder verzweigte C1- bis C8-AlkylGruppe ist.

10. Derivat nach einem der vorstehenden Ansprüche zur Verwendung dieses nach einem der vorstehenden Ansprüche oder zur Verwendung nach einem der vorstehenden Ansprüche, ferner umfassend mindestens ein Derivat des Sinapoylmalats der Formel (VIII): 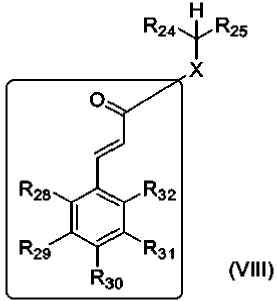 wobei
R₂₄ eine Gruppe (CH₂)ₙCOOH oder (CH₂)ₙN(R₂₇)₃⁺X₂⁻ ist
R₂₅ eine Gruppe H, (CH₂)ₙCOOH, (CH₂)ₙCH₃, (CH₂)ₙ(CHOH)ₙCOOH, (CH₂)ₙ(CHOR₂₆)ₙCOOH, (CH₂)ₙN(R₂₇)₃⁺X₂⁻, (CH₂)ₙ(CHOH)ₙN(R₂₇)₃⁺X₂⁻ oder (CH₂)ₙ(CHOR₃)N(R₂₇)₃⁺X₂⁻ ist
R₂₆ der Gruppe A entspricht
R₂₇ ausgewählt ist aus H oder einer CₙH₂ₙ₊₁-Gruppe
X₁ eine O- oder NH-Gruppe ist
X₂⁻ ein negatives Gegenion (Anion) ist, das aus Halogeniden, Acetaten, Nitraten, Oxiden, Phosphaten, Sulfaten, Bisulfiten, Carboxylaten, und Citraten ausgewählt ist,
n eine ganze Zahl zwischen 0 und 5 ist
R₂₈, R₂₉, R₃₀, R₃₁ und R₃₂ unabhängig ausgewählt sind aus H, OH, NH₂, SH, einem Halogen, einer C₁- bis C₈-Alkyl-Gruppe, die linear, cyclisch oder verzweigt, gesättigt oder ungesättigt ist, einer C₁- bis C₈-O-Alkyl-Gruppe, die linear, cyclisch oder verzweigt, gesättigt oder ungesättigt ist, einer C₁- bis C₈-NH-Alkyl-Gruppe, die linear, cyclisch oder verzweigt, gesättigt oder ungesättigt ist; einer C₁- bis C₈-N-(Alkyl)₂-Gruppe, die linear, cyclisch oder verzweigt, gesättigt oder ungesättigt ist.

11. Derivat nach einem der vorstehenden Ansprüche zur Verwendung dieses nach einem der vorstehenden Ansprüche oder zur Verwendung nach einem der vorstehenden Ansprüche, wobei das Derivat der Meldrumsäure aus den folgenden Verbindungen ausgewählt ist: 5-(2-Furanylmethylen)-2,2-dimethyl-1,3-dioxan-4,6-dion (Verbindung 1), 5-[(4,5-Dimethyl-2-furanyl)methylen]-2,2-dimethyl-1,3-dioxan-4,6-dion (Verbindung 2), 5-[(5-Ethyl-2-furanyl)methylen]-2,2-dimethyl-1,3-dioxan-4,6-dion (Verbindung 3), 5-[[5-(Hydroxymethyl)-2-furanyl]methylen]-2,2-dimethyl-1,3-dioxan-4,6-dion (Verbindung 4), 5-(4-Hydroxy-3-methoxyphenyl)-2,2-dimethyl-1,3-dioxan-4,6-dion (Verbindung 8), 5-(4-Hydroxy-3,5-dimethoxyphenyl)-2,2-dimethyl-1,3-dioxan-4,6-dion (Verbindung 11), 5-(1H-pyrrol-2-ylmethyliden)-2,2-dimethyl-1,3-dioxan-4,6-dion (Verbindung 12), 5-[(1-Methyl)-pyrrol-2-ylmethyliden]-2,2-dimethyl-1,3-dioxan-4,6-dion (Verbindung 13), 2,2-Dimethyl-5-(3-phenyl-2-propenyliden)-1,3-dioxan-4,6-dion (Verbindung 16), 2,2-Dimethyl-5-(3-(4-acetoxy-3-methoxyphenyl)-2-propenyliden)-1,3-dioxan-4,6-dion (Verbindung 17) und 5-[3-(2-Furanyl)-2-propen-1-yliden]-2,2-dimethyl-1,3-dioxan-4,6-dion (Verbindung 19).

## Claims

1. Meldrum's acid derivative of formula (I) 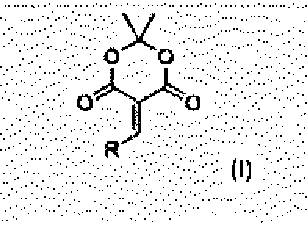
where R is a furan, phenol, pyrrole, vinylphenol, or vinylfuran group,
for use thereof on the skin as a UVA and blue light filter.

2. Use of at least one Meldrum's acid derivative of formula (I) 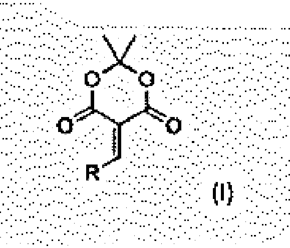
where R is a furan, phenol, pyrrole, vinylphenol, or vinylfuran group,
as a UVA and blue light filter in phytosanitary compositions, in cold protection compositions, in polymers, or in packaging.

3. Derivative according to claim 1 for the use thereof according to claim 1 or use according to claim 2, wherein said furan group R corresponds to the formula (II) 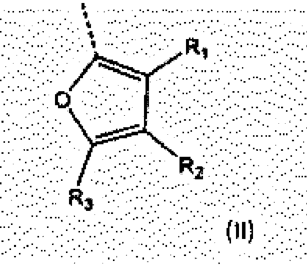 where:
R1 represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₁ and R₂ can be combined to form a -(CH₂)ₙ- or -(CH)ₙ- ring with n ranging from 4 to 8
R₂ represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₁ and R₂ can be combined to form a -(CH₂)ₙ- or -(CH)ₙ- ring with n ranging from 4 to 8
R₂ and R₃ can be combined to form a -(CH₂)ₙ- or -(CH)ₙ- ring with n ranging from 4 to 8
R₃ represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group
R₂ and R₃ can be combined to form a -(CH₂)ₙ- or -(CH)ₙ- ring with n ranging from 4 to 8.

4. Derivative according to claim 1 for the use thereof according to claim 1 or use according to claim 2, wherein said phenol group R corresponds to the formula (III) 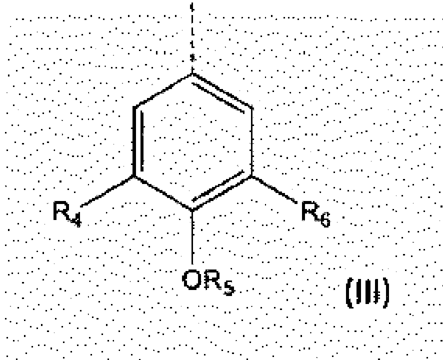 where:
R₄ and R₆ are independently selected from an H, or a linear, cyclic, or branched, saturated or unsaturated C₁ to Cs alkyl group, or a linear, cyclic, or branched, saturated or unsaturated C₁ to C₈ O-alkyl group
R₅ is an H, an acetyl group, or a linear, cyclic, or branched, saturated or unsaturated C₁ to C₈ alkyl group, a silyl, epoxide, benzyl or benzoyl protecting group.

5. Derivative according to claim 1 for the use thereof according to claim 1 or use according to claim 2, wherein said pyrrole group R corresponds to the formula (IV) 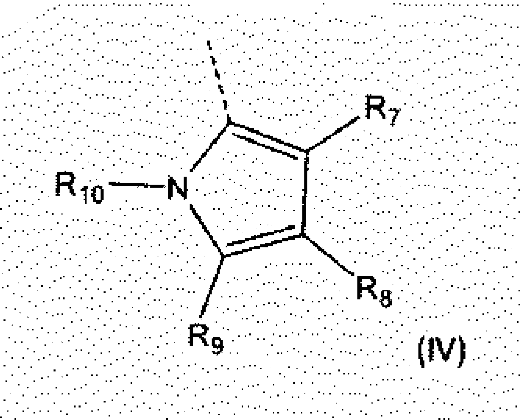 where:
R₇ represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₇ and R₈ can be combined to form a -(CH₂)ₙ- or -(CH)ₙ- ring with n ranging from 4 to 8
R₈ represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₇ and R₈ can be combined to form a -(CH₂)ₙ- or -(CH)ₙ- ring with n ranging from 4 to 8
R₈ and R₉ can be combined to form a -(CH₂)ₙ- or -(CH)ₙ- ring with n ranging from 4 to 8
R₉ represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₈ and R₉ can be combined to form a -(CH₂)ₙ- or -(CH)ₙ- ring with n ranging from 4 to 8
R₉ and R₁₀ can be combined to form a -(CH₂)ₙ- or -(CH)ₙ- ring with n ranging from 4 to 8
R₁₀ represents:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group
R₉ and R₁₀ can be combined to form a -(CH₂)ₙ- or -(CH)ₙ- ring with n ranging from 4 to 8.

6. Derivative according to claim 1 for the use thereof according to claim 1 or use according to claim 2, wherein said vinylphenol group R corresponds to the formula (V) 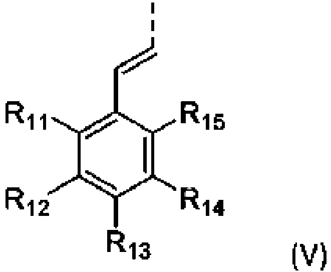 where:
R₁₁, R₁₂, R₁₃, R₁₄, R₁₅ are independently selected from:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₁₁ and R₁₂ can be combined to form a -(CH₂)ₙ- or -(CH)ₙ- ring with n ranging from 4 to 8
R₁₂ and R₁₃ can be combined to form a -(CH₂)ₙ- or -(CH)ₙ- ring with n ranging from 4 to 8
R₁₃ and R₁₄ can be combined to form a -(CH₂)ₙ- or -(CH)ₙ- ring with n ranging from 4 to 8
R₁₄ and R₁₅ can be combined to form a -(CH₂)ₙ- or -(CH)ₙ- ring with n ranging from 4 to 8.

7. Derivative according to claim 1 for the use thereof according to claim 1 or use according to claim 2, wherein said vinylfuran group R corresponds to the formula (VI) 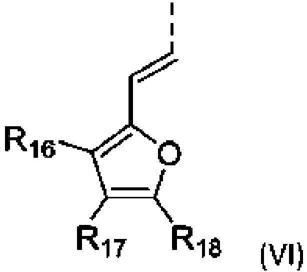 where:
R₁₆, R₁₇, R₁₈ are independently selected from:
H, OH, CHO, CO₂H, NH₂, a cyano group, a halogen
a linear, cyclic, or branched, saturated or unsaturated (CH₂)ₓOH, or (CH₂)ₓNH₂, or (CH₂)ₓCO, or (CH₂)ₓCOOH group, with x ranging from 1 to 30
a linear, cyclic, or branched, saturated or unsaturated C₁ to C₃₀ alkyl group
an amine of the NHR, NR₂, or NRR' type, with R and R' independently selected from a linear, cyclic, or branched, saturated or unsaturated C₁ to C₃₀ alkyl group
an ester bearing a linear, cyclic, or branched, saturated or unsaturated C₁ to C₃₀ alkyl group, or a benzoyl group
an amide bearing a linear, cyclic, or branched, saturated or unsaturated C₁ to C₃₀ alkyl group
a ketone bearing a linear, cyclic, or branched, saturated or unsaturated C₁ to C₃₀ alkyl group
an ether bearing a linear, cyclic, or branched, saturated or unsaturated C₁ to C₃₀ alkyl group, or a silyl, epoxide, or benzyl group;
R₁₆ and R₁₇ can be combined to form a -(CH₂)ₙ- or -(CH)ₙ- ring with n ranging from 4 to 8
R₁₇ and R₁₈ can be combined to form a -(CH₂)ₙ- or -(CH)ₙ- ring with n ranging from 4 to 8.

8. Derivative according to any of claims 1 to 7 for the use thereof according to any of claims 1 to 7 or use according to any of claims 1 to 7, comprising at least two Meldrum's acid derivatives as defined in claim 1 or 2 selected from (i) a derivative substituted with a furan group, (ii) a derivative substituted with a phenol group, and (iii) a derivative substituted with a pyrrole group, (iv) a derivative substituted with a vinylphenol group, and (v) a derivative substituted with a vinylfuran group.

9. Derivative according to any of the preceding claims for the use thereof according to any of the preceding claims or use according to any of the preceding claims, further comprising at least one β-β dimer of formula (VII): 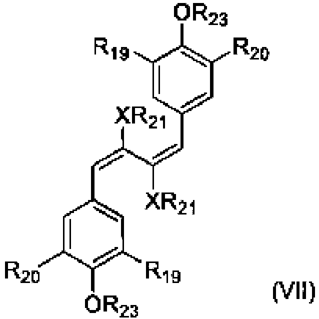 where:
R₁₉ and R₂₀ are independently selected from a linear, cyclic, or branched C1 to C8 alkyl group, or a linear, cyclic, or branched C1 to C8 O-alkyl group;
R₂₁ is an H, a linear, cyclic, or branched C1 to C30 alkyl group, or an organic (mono- or di-)acid hydro group;
R₂₃ is an H, an acetyl group, or a linear, cyclic, or branched C1 to C8 alkyl group, a silyl, benzyl, or benzoyl protecting group;
X is a C=O group, a CO2 group, or a C(O)NR₂₂ group where R₂₂ is an H or a linear, cyclic, or branched C1 to C8 alkyl group.

10. Derivative according to any of the preceding claims for the use thereof according to any of the preceding claims or use according to any of the preceding claims, further comprising at least one sinapoyl malate derivative of formula (VIII): 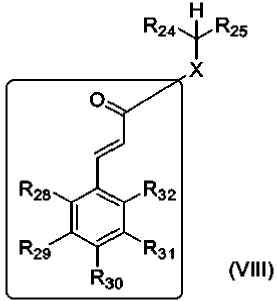 where
R₂₄ is a (CH₂)ₙCOOH or (CH₂)ₙN(R₂₇)₃⁺X₂⁻ group
R₂₅ is an H, (CH₂)ₙCOOH, (CH₂)ₙCH₃, (CH₂)ₙ(CHOH)ₙCOOH, (CH₂)ₙ(CHOR₂₆)ₙCOOH, (CH₂)ₙN(R₂₇)₃⁺X₂⁻, (CH₂)ₙ(CHOH)ₙN(R₂₇)₃⁺X₂⁻, or (CH₂)ₙ(CHOR₃)N(R₂₇)₃⁺X₂⁻ group
R₂₆ corresponds to group A
R₂₇ is selected from H or a CₙH₂ₙ₊₁ group
X₁ is an O or NH group
X₂⁻ is a negative counterion (anion) selected from halides, acetates, nitrates, oxides, phosphates, sulfates, bisulfites, carboxylates, citrates
n being an integer between 0 and 5
R₂₈, R₂₉, R₃₀, R₃₁ and R₃₂ are independently selected from an H, an OH, an NH₂, an SH, a halogen, a linear, cyclic, or branched, saturated or unsaturated C₁ to C₈ alkyl group, a linear, cyclic, or branched, saturated or unsaturated C₁ to C₈ O-alkyl group, a linear, cyclic, or branched, saturated or unsaturated C₁ to C₈ NH-alkyl group; a linear, cyclic, or branched, saturated or unsaturated C₁ to C₈ N-(alkyl)₂ group.

11. Derivative according to any of the preceding claims for the use thereof according to any of the preceding claims or use according to any of the preceding claims, wherein said Meldrum's acid derivative is one of the following compounds: 5-(2-furanylmethylene)-2,2-dimethyl-1,3-dioxane-4,6-dione (compound 1), 5-[(4,5-dimethyl-2-furanyl)methylene]-2,2-dimethyl-1,3-dioxane-4,6-dione (compound 2), 5-[(5-ethyl-2-furanyl)methylene]-2,2-dimethyl-1,3-dioxane-4,6-dione (compound 3), 5-[[5-(hydroxymethyl)-2-furanyl]methylene]-2,2-dimethyl-1,3-dioxane-4,6-dione (compound 4), 5-(4-hydroxy-3-methoxyphenyl)-2,2-dimethyl-1,3-dioxane-4,6-dione (compound 8), 5-(4-hydroxy-3,5-dimethoxyphenyl)-2,2-dimethyl-1,3-dioxane-4,6-dione (compound 11), 5-(1H-pyrrole-2-ylmethylidene)-2,2-dimethyl-1,3-dioxane-4,6-dione (compound 12), 5-[(1-methyl)-pyrrole-2-ylmethylidene]-2,2-dimethyl-1,3-dioxane-4,6-dione (compound 13), 2,2-dimethyl-5-(3-phenyl-2-propenylidene)-1,3-dioxane-4,6-dione (compound 16), 2,2-dimethyl-5-(3-(4-acetoxy-3-methoxyphenyl)-2-propenylidene)-1,3-dioxane-4,6-dione (compound 17), and 5-[3-(2-furanyl)-2-propen-1-ylidene]-2,2-dimethyl- 1,3-dioxane-4,6-dione (compound 19).
